Europäisches Patentamt

European Patent Office

Office européen des brevets

(11) Publication number: **0 326 616**
**A1**

# EUROPEAN PATENT APPLICATION

published in accordance with Art. 158(3) EPC

(21) Application number: **87906212.3**

(22) Date of filing: **25.09.87**

Data of the international application taken as a basis:

(86) International application number:
**PCT/JP 87/00699**

(87) International publication number:
**WO 88/02235 (07.04.88 88/8)**

(30) Priority: **29.09.86 JP 228013/86**

(43) Date of publication of application: **09.08.89**
**Bulletin 89/32**

(84) Designated Contracting States: **BE CH DE FR GB IT LI NL SE**

(51) Int. Cl.⁴: **A 61 B 5/02**

(71) Applicant: **Terumo Kabushiki Kaisha, No. 44-1, Hatagaya 2-chome Shibuya-ku, Tokyo 151 (JP)**

(72) Inventor: **MORIUCHI, Yousuke, Terumo K. K. Technical R & D Division 2656-1, Ohbuchi§Fuji-shi§Shizuoka-ken 417 (JP)**
Inventor: **ISHIDA, Toshinobu, Terumo K. K. Technical R & D Division 2656-1, Ohbuchi Fuji-shi Shizuoka-ken 417 (JP)**

(74) Representative: **Henkel, Feiler, Hänzel & Partner, Möhlstrasse 37, D-8000 München 80 (DE)**

(54) **DOME FOR LIQUID PRESSURE TRANSDUCERS.**

㋕ The bottom of a housing (21) is opened in the downward direction, and a diaphragm (25) is sticked to the open end surface thereof. A pressure chamber (24) defined by the inner surface of the housing (21) and the diaphragm (25) is formed. The housing (21) is provided with a liquid inlet port (22) and a liquid outlet port (23) which cummunicate with the pressure chamber (24). A locking projection provided on the inner surface of the upper end portion of an cylindrical connector (26) is engaged with a locking groove (34) provided in the outer circumferential surface of a side portion of the housing (21) so that the connector (26) can be rotatably mounted to the housing (21). A female screw is provided in the inner surface of the connector (26). When this connector (26) is turned, a transducer (31) which is provided with a male screw on the outer surface thereof is screwed. As a result, the transducer (31) is combined with the housing (21) with a pressure-receiving plate (32) being in contact with the diaphragm (25).

- 1 -

SPECIFICATION

Pressure dome

[Field of the Invention]

The present invention relates to a pressure dome for a liquid pressure transducer used in, for example, a monitoring type blood pressure measuring system.

[Background Art]

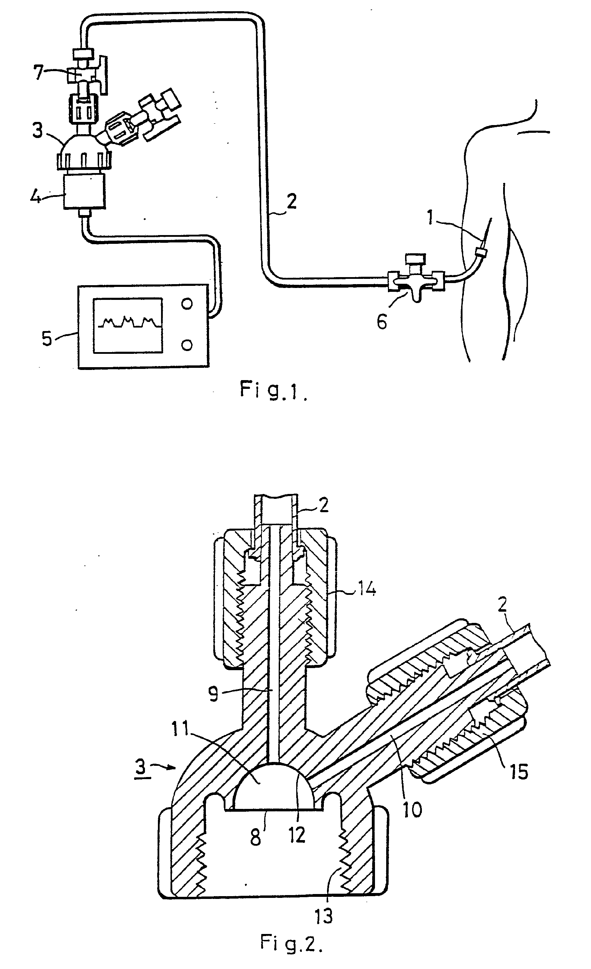

Where it is necessary to monitor accurately the blood pressure of a patient as in, for example, the analgesic surgical operation, used is a monitoring type blood pressure measuring system as shown in Fig. 1.

In the system shown in Fig. 1, the proximal end of a catheter 1 whose distal end is retained within a blood vessel of a patient is connected to a pressure transducer 4, with a pressure transmitting tube 2 and a pressure dome 3 interposed therebetween. The tube 2 and dome 3 are filled with, for example, a physiological saline solution which serves to transmit the blood pressure of the patient to a diaphragm provided within the pressure dome 3. The pressure received by the diaphragm is further transmitted to the transducer 4 so as to be converted into an electric signal. Further, a display device 5, e.g., a CRT, and a recording device are connected to the transducer 4, with the result that the blood pressure of the patient can be recorded while directly monitoring the blood pressure of the patient all the time.

Fig. 2 is a cross sectional view showing the construction of the conventional pressure dome 3 included in the system shown in Fig. 1. It is seen that a pressure chamber 11 defined by a hemispherical wall 12 is provided within the dome 3. A diaphragm 8 attached to the bottom of the pressure chamber 11 in a liquid-tight fashion serves to detect the blood pressure transmitted through the physiological saline solution. The solution introduced into the pressure chamber 11 through an inlet port 9 is discharged through an outlet port 10. The peripheral portion of the dome 3 extends downward to form a sleeve acting as a coupling portion. The sleeve is internally threaded for coupling with the pressure transducer 4 which is externally threaded. When the transducer 4 is engaged with the dome 3, a pressure-receiving plate mounted at the tip of the transducer 4 is in direct contact with the diaphragm 8. As seen from the drawing, the pressure dome 3 including the coupling portion with the transducer is formed integrally. It is also seen that the pressure transmitting tubes 2 are fixed by internally threaded members 14 and 15.

In the blood pressure measuring system shown in Fig. 1, it is necessary to completely discharge the air from within the pressure transmitting mechanism. Since the air can be compressed greatly, the presence of air within the pressure transmitting mechanism inhibits a satisfactory blood pressure transmission to the diaphragm 8. In addition, the frequency characteristics are fluctuated by the presence of air. It follows that the presence of air makes it impossible to measure the blood pressure accurately. Thus, a priming operation is performed in order to discharge the air completely from within the pressure transmitting mechanism including the catheter 1, pressure transmitting tube 2 and pressure dome 3 before the catheter 1 is inserted into a blood vessel of a patient. The priming operation is performed by injecting a physiological saline solution into the catheter 1 and the pressure dome 3 through a three-port cock 6 mounted to the pressure transmitting tube 2 until the injected solution overflows through the distal end of the catheter 1 and the outlet port 10 of the dome 3.· After the pressure transmitting mechanism has been completely filled with the physiological saline solution by the priming operation, the blood pressure of the patient is measured as shown in Fig. 1.

As described previously, the conventional pressure dome included in the monitoring type blood pressure measuring system comprises a coupling portion with a transducer. The coupling portion is formed integrally with the dome, giving rise to several problems.

First of all, it is difficult to attach the diaphragm 8 to the bottom of the pressure chamber 11. The diaphragm 8 is attached by using an adhesive or by means of thermal fusion. Naturally, the diaphragm is inserted from the open end of the coupling portion with the transducer for the attachment. What should be noted is that the attaching portion is recessed inward from the open end of the coupling portion, and that the open end of the coupling portion is very small, leading to a very troublesome attaching operation. In addition, the attached diaphragm fails to exhibit a constant quality, causing deterioration of the performance.

What should also be noted is that it is necessary to rotate the pressure dome 3 or the pressure transducer 4 for achieving the coupling. However, it is very difficult to perform the rotation, because a long cord is connected to the transducer 4 and the pressure transmitting tube 2 is connected to the pressure dome 3. Further, the diaphragm 8 and the pressure-receiving plate of the transducer tend to be damaged because of accompanying the mutual rotation in the engaging step, leading to a low performance.

An additional difficulty to be noted is that it is impossible to completely discharge the air between the diaphragm 8 and the pressure-receiving plate of the transducer in the step of engagement between the pressure dome 3 and the transducer 4, with the result that the pressure-receiving plate fails to be contacted sufficiently to the diaphragm 8. To avoid the problem, employed is a wet coupling method in which the pressure-receiving plate having water droplets attached to the surface thereof is brought into contact with the diaphragm 8 such that the pressure can be transmitted sufficiently through the water. In this case, however, the attached water droplets tend to drop out because of rotation of the transducer during the engagement, resulting in failure to achieve the desired object.

[Disclosure of the Invention]

The present invention is intended to provide a pressure dome for a liquid pressure transducer, said dome being constructed such that the diaphragm can be readily attached and the quality of the attached diaphragm can be improved, that the pressure transmission between the diaphragm and the pressure-receiving plate can be improved, and that the transducer can be readily inserted for the screw engagement.

The pressure dome for a liquid pressure transducer according to the present invention comprises a housing open at the bottom, a pressure chamber defined by the inner surface of the housing and a diaphragm mounted in a liquid-tight fashion at the bottom of the housing, a liquid inlet port and a liquid outlet port formed in the housing to communicate with the pressure chamber, and a cylindrical coupling member rotatably engaged with the outer surface of the lower portion of the housing, and is characterized in that a pressure transducer is inserted into the dome by rotating the coupling member such that a pressure-receiving plate at the tip of the transducer is in direct contact with the diaphragm.

In the present invention, the diaphragm is formed in general of a hydrophobic resin such as polyethylene, polypropylene, polytetrafluoroethylene or polyvinylidene fluoride.

The housing and the coupling member, which are formed as separate members, are joined to each other by the so-called "forced coupling". Thus, it is desirable for at least the coupling member to be formed of an elastic resin. Also, the pressure dome used in a monitoring type blood pressure measuring system is generally of disposable type. In this case, it is desirable in view of the manufacturing cost to use a synthetic resin for forming each of the housing and the coupling member.

In the present invention, the housing serving to define the pressure chamber should desirably be of a hemispherical shape.

It is important to note that a diaphragm can be readily mounted to the dome because the diaphragm is mounted before the coupling member is joined to the dome. Also, a pressure transducer can be readily coupled with the dome without doing damage to the diaphragm in the coupling step. Further, the dome of the particular construction permits improving the pressure transmitting characteristics.

[Brief Description of the Drawings]

Fig. 1 schematically shows a monitoring type blood pressure measuring system using a pressure transducer;

Fig. 2 is a cross sectional view showing the construction of the conventional pressure dome for a liquid pressure transducer included in the system shown in Fig. 1;

Fig. 3 is a front view, partly broken away, showing a pressure dome for a liquid pressure transducer according to one embodiment of the present invention; and

Fig. 4 shows how to join a pressure transducer to the dome shown in Fig. 3.

[Best Mode of Embodiment]

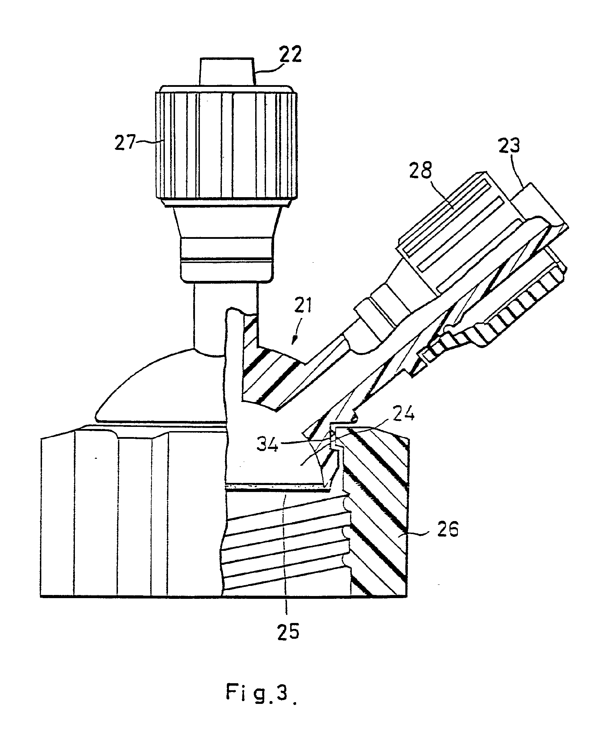

Fig. 3 shows a pressure dome for a liquid pressure transducer according to one embodiment of the present invention. It is seen that the dome comprises a housing 21 having a hemispherical pressure chamber 24 formed therein. The pressure chamber 24 is defined by the inner surface of the housing 21 except for the bottom. The housing 21 is provided with a liquid inlet port 22 and a liquid outlet port 23 each communicating with the pressure chamber. Further, a diaphragm 25 is attached in a liquid-tight fashion to the open lower end of the housing 21 to provide the bottom of the pressure chamber 24.

Reference numeral 26 denotes a coupling member with a transducer. The coupling member 26, which is substantially cylindrical, is internally threaded for screw engagement with a transducer. The upper end of the coupling member 26 is inwardly projected to provide an engagement projection, which is engaged with an engagement groove 34 formed around the outer surface of the housing 21. Because of the engagement, the coupling member 26 is joined to the lower part of the housing 21. Also, the coupling member 26, which is engaged with a lower part of the housing 21, cannot be moved in the axial direction, but is rotatable about the housing 21. Incidentally, threaded members 27, 28 for fixing the pressure transmitting tubes 2 shown in Fig. 3 are mounted to the liquid inlet port 22 and the liquid outlet port 23, respectively.

In the embodiment described above, the coupling member 26 is formed separately from the housing 21 and joined to the housing 21 by the so-called "forced coupling". Before the forced coupling step, the diaphragm 25 is attached to the open lower end of the pressure chamber formed in the housing 21. Thus, the attaching operation, which is not obstructed by the coupling member 26, can be performed easily. In addition, it is possible to use various tools in attaching the diaphragm. It follows that it is possible to improve the attached state of the diaphragm and to attach the diaphragm uniformly, leading to an improved pressure transmitting characteristics of the diaphragm.

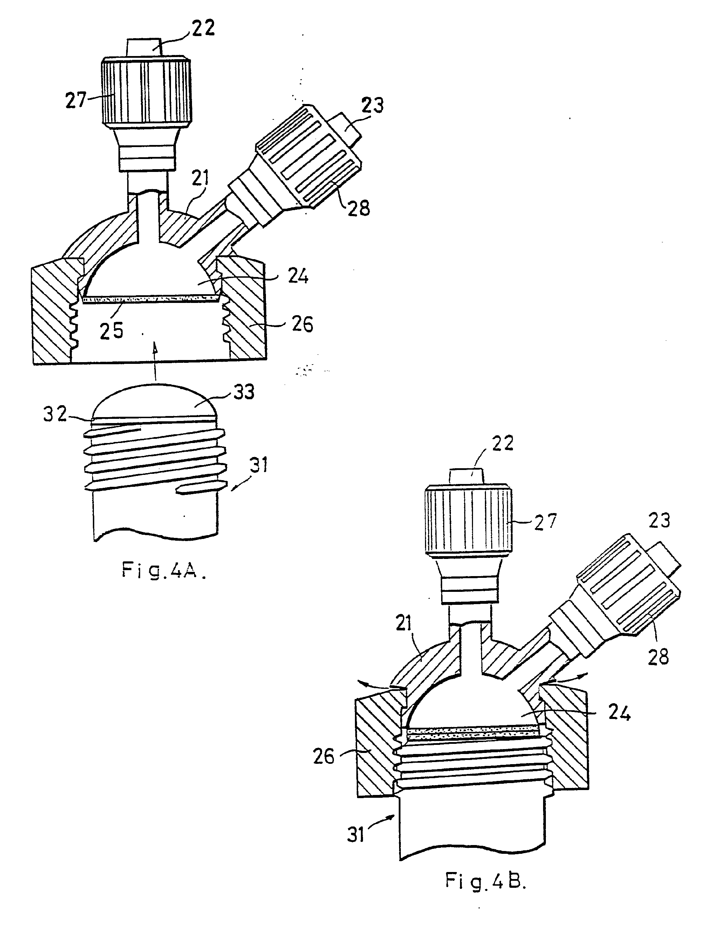

Fig. 4 shows how to join a pressure transducer 31 to the dome of the particular construction described above. As seen from Fig. 4A, the transducer 31 provided with a pressure-receiving plate 32 is externally threaded. A water droplet 33 is attached to the surface of the plate 32. Under the condition shown in Fig. 4A, the transducer 31 is screwed and inserted into the coupling member 26 as denoted by an arrow. It should be noted that the transducer 31 can be screwed by simply rotating the coupling member 26 without rotating the housing 21 of the dome and the transducer 31. Naturally, the cord connected to the transducer 31 is not twisted, markedly facilitating the insertion of the transducer 31. Also, the water droplet 33 does not drop out during the screwing because the transducer 31 is not rotated during the screwing of the transducer 31 into the coupling member 26.

After insertion of the transducer 31, the pressure-receiving plate 32 of the transducer is brought into contact with the diaphragm 25 of the dome with the water droplet 33 interposed therebetween, as shown in Fig. 4B, and coupling of pressure transmission property is attained between the pressure plate and diaphragm. During the insertion, the air within the coupling member 26 is released to the outside through a clearance between the housing 24 and the coupling member 26, as denoted by arrows in Fig. 4B. In other words, the clearance between the diaphragm 25 and the pressure-receiving plate 32 is filled with water, resulting in a marked improvement in the efficiency of pressure transmission from the diaphragm 25 to the pressure-receiving plate 32.

What should also be noted is that the pressure-receiving plate 32 is not rotated relative to the diaphragm 25 for achieving the mutual contact.
Naturally, the diaphragm is not damaged by the friction
between the two.

Claims:

1. A pressure dome for a liquid pressure transducer comprising a housing open at the bottom, a pressure chamber defined by the inner surface of the housing except for the bottom, a diaphragm mounted in a liquid-tight fashion at the bottom of the housing, a liquid inlet port and a liquid outlet port formed in the housing to communicate with the pressure chamber, and a coupling member for bringing the pressure-receiving plate of a pressure transducer into contact with the diaphragm, characterized in that the coupling member is formed separately from the housing and engaged with the outer surface of the lower portion of the housing and that a clearance is provided between the coupling member and the housing.

2. The dome according to claim 1, characterized in that the coupling member is internally threaded for engagement with the transducer and is rotatable about the axis of the housing.

3. The dome according to claim 1, characterized in that the pressure chamber is hemispherical.

4. The dome according to claim 1 or 2, characterized in that at least the coupling member is formed of an elastic material.

5. The dome according to claim 1, characterized in that the diaphragm is formed of a hydrophobic resin selected from the group consisting of polyethylene, polypropylene, polytetrafluoroethylene and polyvinylidene fluoride.

Fig.1.

Fig.2.

Fig.3.

Fig.4A.

Fig.4B.

# INTERNATIONAL SEARCH REPORT

**0 326 616**

International Application No    PCT/JP87/00699

## I. CLASSIFICATION OF SUBJECT MATTER (if several classification symbols apply, indicate all)

According to International Patent Classification (IPC) or to both National Classification and IPC

Int.Cl$^4$    A61B5/02

## II. FIELDS SEARCHED

### Minimum Documentation Searched [4]

| Classification System | Classification Symbols |
|---|---|
| IPC | A61B5/02 |

### Documentation Searched other than Minimum Documentation to the Extent that such Documents are Included in the Fields Searched [5]

| | |
|---|---|
| Jitsuyo Shinan Koho | 1960 – 1987 |
| Kokai Jitsuyo Shinan Koho | 1971 – 1987 |

## III. DOCUMENTS CONSIDERED TO BE RELEVANT [14]

| Category* | Citation of Document, [16] with indication, where appropriate, of the relevant passages [17] | Relevant to Claim No. [18] |
|---|---|---|
| Y | JP, A, 60-88535 (American Hospital Supply Corporation) 18 May 1985 (18. 05. 85) Page 4, upper left column, line 5 to page 5, upper right column, line 7 & US, A, 4539349 & EP, A2, 138397 | 1-5 |
| Y | JP, Y2, 53-18711 (Dia Medical System Kabushiki Kaisha) 18 May 1978 (18. 05. 78) Page 1, right column, lines 22 to 23 (Family: none) | 1-5 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance, the claimed invention cannot be considered novel or cannot be considered to involve an inventive step

"Y" document of particular relevance, the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search [2] | Date of Mailing of this International Search Report [2] |
|---|---|
| December 2, 1987 (02.12.87) | December 14, 1987 (14.12.87) |

| International Searching Authority [1] | Signature of Authorized Officer [2] |
|---|---|
| Japanese Patent Office | |

Form PCT ISA 210 (second sheet) (October 1977)